# EUROPEAN PATENT APPLICATION

(11) **EP 4 001 886 A1**
(43) Date of publication of application: **25.05.2022**
(21) Application number: 21208622.7
(22) Date of filing: 16.11.2021
(51) Int. Cl.: G01N 1/22, B01L 7/00

(54) **SYSTEMS AND METHODS TO OBTAIN A BIOLOGICAL SAMPLE REPRESENTATIVE OF A PASSENGER CABIN ON AN AIRCRAFT AUTOMATICALLY FROM THE COLLECTOR DEVICE**

(30) Priority: 16.11.2020 US 202063114330 P; 16.11.2020 US 202063114339 P; 16.11.2020 US 202063114350 P; 16.11.2020 US 202063114400 P; 16.11.2020 US 202063114064 P; 16.11.2020 US 202063114157 P; 16.11.2020 US 202063114386 P; 16.11.2020 US 202063114366 P
(71) Applicant: Koninklijke Fabriek Inventum B.V., 3439 MG Nieuwegein (NL)
(72) Inventor: GONZALEZ, Arnau Castillo, 3607 AK Maarssen (NL); SURAWSKI, Eric, Hebron, Connecticut 06248 (US)
(74) Representative: Dehns

(57) **Abstract**

A system for monitoring aircraft air including a collector (102) for collecting particulate samples positioned within at least one of an outlet flow path or a recirculation flow path (104) of an environmental control system of an aircraft, wherein the collector is accessible to a user within the cabin of the aircraft.

## Description

### CROSS-REFERENCE TO RELATED APPLICATION

The following application claims priority to U.S. Provisional Patent Applications with the following Serial Nos. 63/114,330, 63/114,339, 63/114,350, 63/114,400, 63,114,064, 63/114,157, 63/114,386, 63/114,366 all filed on November 16, 2020.

### BACKGROUND OF THE INVENTION

### 1. Field of the Invention

The present application is related to a system and method used collect a representative air sample of an aircraft, more specifically to a method and systems for collecting a biological sample from within the aircraft.

### 2. Description of Related Art

The spread progression of SARS-CoV-2 around the world has risen a red flag: Economic economic globalization creates systemic risks. As trade, finance, travel, cyber and other networks grow in scale and interact, they become more complex and unstable. The transporters of the goods of the global economy, such as major airport hubs, are also spreaders of the pathogens. The 2008 global financial crisis provided a dramatic example of how contagions could spread from the US to global markets overnight. So too has the rapid spread of cyber viruses. In health, rising life expectancy and success in preventing a repeat of the devastating influenza pandemic of 1918, which infected about one-third of the world's population and killed as many as 50m people, has created a false sense of security. But the world is now more interdependent. For example, China represents almost one-fifth of global output, is integral to global supply chains, and its tourists spend over $260 billion annually. The COVID-19 pandemic shed light on the need for better monitoring, detecting, and isolating ill passengers, specifically due to the havoc that was wreaked detrimental impact on the global economy, specifically air travel due to closed borders, movement restrictions, and testing requirements.

However, the COVID-19 pandemic the air travel industry has proven that air travel can be safe and that aircraft cabins have a well-managed airflow that inhibits minimize the risk for transmission of virus, and that being seated onboard an aircraft is safer than shopping in large stores. Governments and other authorities need to assume that aircraft are contaminated until proven "clean", as 25% of COVID-19 cases are asymptomatic or pre-symptomatic; but still contagious. Thus, if borders shutdown and there is a drastic reduction in international travel, global passenger travel is greatly reduced. To date travelers and governments have relied on individual diagnostic tests. The uncertainty of the results has reduced people's inclination to travel and subsequent airline inclination to maintain routes.

Accordingly, there is still a need in the art for virus and pathogen detection systems and methods. The present disclosure provides a solution for providing a test and analyzing a test aboard an aircraft.

### SUMMARY OF THE INVENTION

A system for monitoring aircraft air is disclosed. The system includes a collector for collecting particulate samples positioned within at least one of an outlet flow path or a recirculation flow path of an environmental control system of an aircraft, wherein the collector is accessible to a user within the cabin of the aircraft. The collector is out of line of sight view of cabin crew while in a collecting position. The collector becomes accessible by a lift, an access gate, or a roller to the user within the cabin of the aircraft. The particulate samples include droplets exhaled from passengers throughout a duration of a flight. The collector can include a filter material. The collector can include an adaptor and a filter material operatively connected to the adaptor. The adapter can include a frame and the filter material is mounted to the frame. It is also considered that the collector can be portable within the cabin of the aircraft.

The system can also include a mounting slot in the outlet flow path upstream from the outflow valve, wherein the collector is positioned within the mounting slot. The collector can be configured and adapted to be removed from the mounting slot for testing by using a lift or roller.

A method of collecting particulates from aircraft air is also disclosed. The method includes capturing particulates in an outlet flow path with a collector for a period of time, wherein capturing particulates is unaided by a pump in order to move the air to the collector, removing the collector from at least one of the outlet flow path or the recirculation flow path for testing, inserting a clean collector into at least one of the outlet flow path or the recirculation flow path for use during another period of time. The recirculation flow path can be based on inherent pressure differences within the aircraft cabin, galleys, and cargo bay. The method can include doing a Polymerase Chain Reaction (PCR) test on at least one particulate captured in the collector, and relaying a result of the PCR test to a central data center, wherein the period of time is a duration of a flight, and wherein the PCR test is done on-board an aircraft after the duration of the flight to determine if the aircraft is virus free upon arrival. The collector can include an adaptor and a filter material operatively connected to the adaptor, the method further comprising removing the filter material from the adaptor. The adapter can be cleaned using a solution containing isopropyl alcohol.

These and other features of the systems and methods of the subject disclosure will become more readily apparent to those skilled in the art from the following detailed description of the preferred embodiments taken in conjunction with the drawings.

### BRIEF DESCRIPTION OF THE DRAWINGS

So that those skilled in the art to which the subject disclosure appertains will readily understand how to make and use the devices and methods of the subject disclosure without undue experimentation, preferred embodiments thereof will be described in detail herein below with reference to certain figures, wherein:

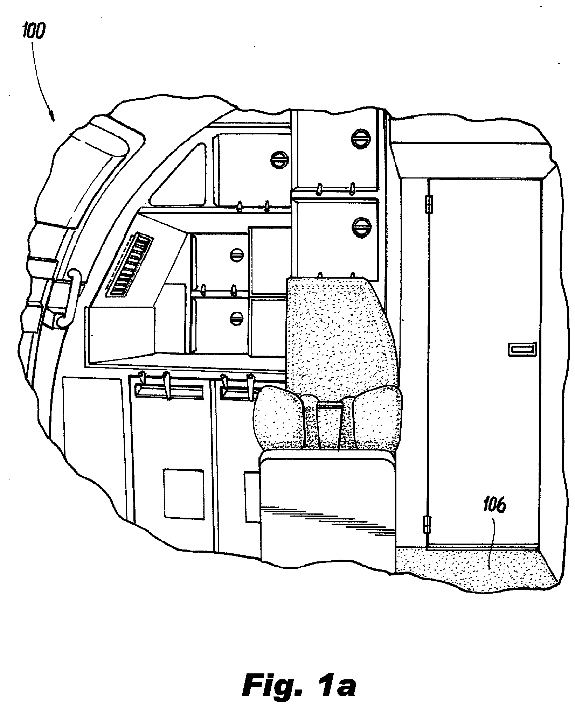

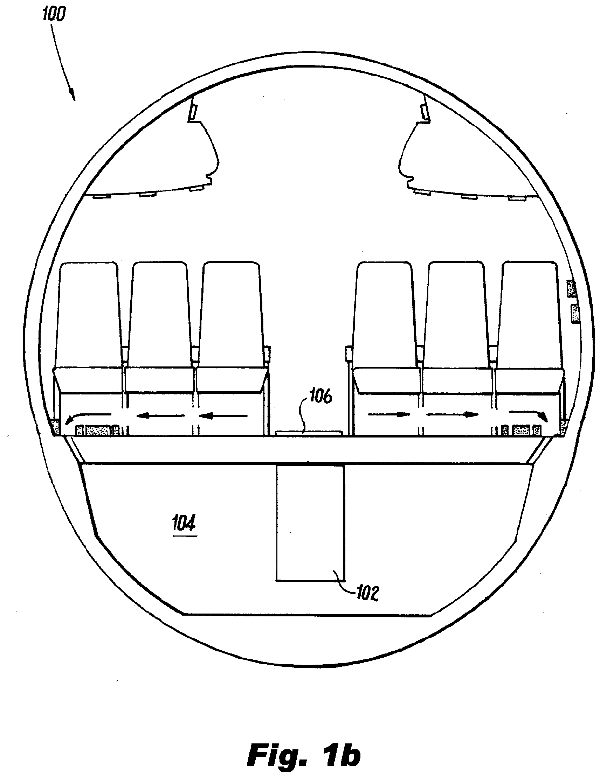

Figs. 1a and 1b show an embodiment of a system for monitoring aircraft air constructed in accordance with the present disclosure, showing a roller or lift placement to bring the collector to the users/

### DETAILED DESCRIPTION OF THE PREFERRED EMBODIMENTS

Reference will now be made to the drawings wherein like reference numerals identify similar structural features or aspects of the subject disclosure. For purposes of explanation and illustration, and not limitation, a schematic view of an exemplary embodiment of a system monitoring aircraft air in accordance with the disclosure showing a collector within a cabin of an aircraft is shown in Figs. 1a and 1b and is designated generally by reference character 100. The systems and methods described herein can be used to provide access to air monitors that are located within the cargo bay. The embodiments of the system for monitoring aircraft air of the present disclosure provide a means for testing air in any enclosed space such as inside an aircraft, and allows for the detection of a virus or other contaminant.

As shown in Figs. 1a and 1b, a system 100 for monitoring aircraft air is disclosed. The system 100 includes a collector 102 for collecting particulate samples positioned within at least one of an outlet flow path 104 or a recirculation flow path of an environmental control system of an aircraft. The collector 102 is accessible to a user within the cabin of the aircraft, but is out of line of sight view of cabin crew while in a collecting position. The collector 102 becomes accessible and visible by a lift, an access gate, or a roller to the user within the cabin of the aircraft, for example, through a trap door 106 on the floor of the galley area that would give access to a receiving box with the sample. Different systems can be used for that transport ranging from a traditional mechanical belt, a vacuum powered transport pipe or a magnetic sling to bring the sample from below the floor to the testing area.

The particulate samples include droplets exhaled from passengers throughout a duration of a flight. The collector 102 includes a filter material which can be scrubbed to remove the collected samples from the collector, alternatively the collector can be removed and replaced by a clean collector prior to the next flight. The collector can include an adaptor and a filter material operatively connected to the adaptor. The adapter can include a frame and the filter material is mounted to the frame.

The system can also include a mounting slot in the outlet flow path upstream from the outflow valve, wherein the collector is positioned within the mounting slot. The collector 102 can be configured and adapted to be removed from the mounting slot for testing by using a lift or roller.

A method of collecting particulates from aircraft air is also disclosed. The method includes capturing particulates in an outlet flow path with a collector for a period of time, wherein capturing particulates is unaided by a pump in order to move the air to the collector, removing the collector from at least one of the outlet flow path or the recirculation flow path for testing, inserting a clean collector into at least one of the outlet flow path or the recirculation flow path for use during another period of time, wherein the collector is located below the cabin area of the aircraft and is brought up via a hatch or rollers at the conclusion of the flight by the cabin crew or other specialty personnel for testing and replacement. The recirculation flow path can be based on inherent pressure differences within the aircraft cabin, galleys, and cargo bay.

The method can include doing a Polymerase Chain Reaction (PCR) test on at least one particulate captured in the collector, and relaying a result of the PCR test to a central data center, wherein the period of time is a duration of a flight, and wherein the PCR test is done on-board an aircraft after the duration of the flight to determine if the aircraft is virus free upon arrival. The collector can include an adaptor and a filter material operatively connected to the adaptor, the method further comprising removing the filter material from the adaptor. The adapter can be cleaned using isopropyl alcohol.

The methods and systems of the present disclosure, as described above and shown in the drawings, provide for a method of operating, using, and replacing a sample collector for testing the air quality and pathogen presence on an aircraft. While the apparatus and methods of the subject disclosure have been shown and described with reference to preferred embodiments, those skilled in the art will readily appreciate that changes and/or modifications may be made thereto without departing from the scope of the invention as defined by the claims.

## Claims

1. A system for monitoring aircraft air comprising:
a collector (102) for collecting particulate samples positioned within at least one of an outlet flow path (104) or a recirculation flow path of an environmental control system of an aircraft, wherein the collector is accessible to a user within the cabin of the aircraft.

2. The system of claim 1, wherein the collector (102) is out of line of sight view of cabin crew while in a collecting position.

3. The system of claim 1 or 2, wherein the collector is accessible by a lift, an access gate, or a roller to the user within the cabin of the aircraft.

4. The system of any preceding claim, wherein the particulate samples include droplets exhaled from passengers throughout a duration of a flight.

5. The system of any preceding claim, wherein the collector includes a filter material, or wherein the collector includes an adaptor and a filter material operatively connected to the adaptor, and optionally wherein the adapter is a frame and the filter material is mounted to the frame.

6. The system of any preceding claim, further comprising a mounting slot in the outlet flow path upstream from the outflow valve, wherein the collector is positioned within the mounting slot.

7. The system as recited in any preceding claim, wherein the collector is configured and adapted to be removed from the mounting slot for testing.

8. A method for collecting particulates from aircraft air comprising:
capturing particulates in an outlet flow path (104) with a collector (102) for a period of time, wherein capturing particulates is unaided by a pump in order to move the air to the collector;
removing the collector from at least one of the outlet flow path or the recirculation flow path for testing; and
inserting a clean collector into at least one of the outlet flow path or the recirculation flow path for use during another period of time.

9. The method of claim 8, wherein the recirculation flow path is based on inherent pressure differences within the aircraft cabin, galleys, and cargo bay.

10. The method of claim 9, further comprising conducting a Polymerase Chain Reaction (PCR) test on at least one particulate captured in the collector.

11. The method of claim 10, further comprising relaying a result of the PCR test to a central data center.

12. The method of claim 10 or 11, wherein the period of time is a duration of a flight, and wherein the PCR test is done on-board an aircraft after the duration of the flight to determine if the aircraft is virus free upon arrival.

13. The method of any of claims 8 to 12, wherein the collector includes an adaptor and a filter material operatively connected to the adaptor, the method further comprising removing the filter material from the adaptor.

14. The method of claim 13, further comprising cleaning the adapter in isopropyl alcohol.
